(19) **Europäisches Patentamt**
**European Patent Office**
**Office européen des brevets**

(11) **EP 3 275 538 B1**

(12) **FASCICULE DE BREVET EUROPEEN**

(45) Date de publication et mention
de la délivrance du brevet:
**24.06.2020 Bulletin 2020/26**

(51) Int Cl.:
***B01J 29/74*** *(2006.01)*   ***C07C 5/27*** *(2006.01)*
***C10G 45/64*** *(2006.01)*

(21) Numéro de dépôt: **17305989.0**

(22) Date de dépôt: **25.07.2017**

(54) **PROCEDE D'ISOMERISATION DE COUPES C8 AROMATIQUES AVEC UN CATALYSEUR COMPRENANT UNE ZEOLITHE IZM-2 AYANT UN RAPPORT MOLAIRE SI/AL OPTIMISE POUR L'ISOMERISATION DE COUPES C8 AROMATIQUES**

VERFAHREN ZUR ISOMERISATION VON AROMATISCHEN C8 FRAKTIONEN MIT EINEM KATALYSATOR ENTHALTEND ZEOLITH IZM-2 MIT EINEM SI/AL VERHÄLTNIS GEEIGNET FÜR DIE ISOMERISATION VON AROMATISCHEN C8 FRAKTIONEN

PROCESS FOR THE ISOMERISATION OF AROMATIC C8 FRACTIONS WITH A CATALYST COMPRISING ZEOLITE IZM-2 HAVING A MOLAR SI/AL RATIO OPTIMISED FOR THE ISOMERISATION OF AROMATIC C8 FRACTIONS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **26.07.2016 FR 1657146**

(43) Date de publication de la demande:
**31.01.2018 Bulletin 2018/05**

(73) Titulaire: **IFP Energies nouvelles**
**92500 Rueil-Malmaison (FR)**

(72) Inventeur: **BOUCHY, Christophe**
**69007 LYON (FR)**

(74) Mandataire: **IFP Energies nouvelles**
**Département Propriété Industrielle**
**Rond Point de l'échangeur de Solaize**
**BP3**
**69360 Solaize (FR)**

(56) Documents cités:
**WO-A1-2010/015733   WO-A1-2013/153317**

## Description

La présente invention se rapporte à un procédé d'isomérisation d'une coupe $C_8$ aromatiques mettant en œuvre un catalyseur comprenant une zéolithe désignée sous l'appellation IZM-2 de rapport molaire silicium sur aluminium optimisé (rapport Si/Al, exprimé en moles de silicium par mole d'aluminium).

## Etat de la technique antérieure

Les sources d'aromatiques à huit atomes de carbone sont principalement issues du procédé de reforming (reformat) et de vapocraquage (essences de pyrolyse). La distribution des aromatiques à huit atomes de carbone au sein de ces coupes est variable : généralement de 10 à 30% d'éthylbenzène avec pour complément les trois isomères de xylènes : le para-xylène, le méta-xylène et l'ortho-xylène. Typiquement la distribution au sein de ce complément de xylènes est de 50% en méta-xylène, 25% en ortho-xylène et 25% en para-xylène. Au sein de ce complément de xylènes, le para-xylène est un isomère particulièrement recherché. En effet ce dernier, par l'intermédiaire du diméthyltéréphtalate et de l'acide téréphtalique, permet la production de fibres polyesters utilisées pour les vêtements et résines et films de polyéthylène téréphtalate (PET). Il est ainsi souhaitable de maximiser la production de para-xylène au détriment des autres aromatiques à huit atomes de carbone. Ceci est réalisé par la mise en oeuvre de procédés catalytiques d'isomérisation. Après extraction du para-xylène, la coupe résiduelle, riche en méta-xylène, ortho-xylène et éthylbenzène est envoyée vers une unité catalytique d'isomérisation qui redonne un mélange d'aromatiques à huit atomes de carbone dans lequel la proportion des xylènes est proche de l'équilibre thermodynamique et la quantité d'éthylbenzène amoindrie grâce à la conversion de l'éthylbenzène. Ce mélange est à nouveau envoyé dans une unité d'extraction du para-xylène et la coupe résiduelle envoyée à l'unité d'isomérisation. On crée ainsi une « boucle $C_8$ aromatiques » qui permet de maximiser la production de para-xylène (E. Guillon, P. Leflaive, Techniques de l'Ingénieur, J5920, V3). On peut mettre en oeuvre une unité d'isomérisation pour isomériser les xylènes en para-xylène et convertir l'éthylbenzène en benzène par la réaction de désalkylation de l'éthylbenzène. Dans ce cas on parle d'isomérisation « désalkylante » de la coupe. La coupe résiduelle peut aussi être envoyée vers une unité catalytique d'isomérisation, pour isomériser les xylènes en para-xylène et convertir l'éthylbenzène en xylènes par la réaction d'isomérisation de l'éthylbenzène. On parle alors d'isomérisation « isomérisante » de la coupe. Ces procédés industriels utilisent généralement des catalyseurs hétérogènes mis en oeuvre en lit fixe et opérant en phase vapeur sous pression d'hydrogène. Ces deux types de procédés se distinguent par les conditions opératoires et par la formulation des catalyseurs mis en oeuvre (par leur nature et/ou leur teneur en fonction hydro-déshydrogénante et/ou en acide). La présente invention s'inscrit dans le domaine de l'isomérisation « isomérisante ».

Dans le cas de l'isomérisation « isomérisante », le catalyseur est de type bifonctionnel et présente à la fois une fonction acide (généralement apportée par au moins une zéolithe) et une fonction hydro-déshydrogénante apportée par un métal noble (généralement le platine). Il a en effet été démontré que l'isomérisation de l'éthylbenzène en xylènes implique un mécanisme de type bifonctionnel. L'éthylbenzène est tout d'abord hydrogéné en éthylcyclohéxènes sur les sites métalliques, ces intermédiaires cyclo-oléfiniques sont ensuite isomérisés en diméthylcyclohéxènes sur les sites acides de Bronsted. Enfin les diméthylcyclohéxènes sont déshydrogénés en xylènes sur les sites métalliques. L'utilisation d'une fonction hydro-déshydrogénante forte telle que le platine induit également la production de cycles naphténiques par hydrogénation des cycles aromatiques correspondant.

En plus des réactions d'isomérisation désirées, il est souhaitable de limiter les réactions parasites de type :

- désalkylation de l'éthylbenzène en benzène et éthylène ;

- dismutation de l'éthylbenzène en diéthylbenzène et benzène, ou des xylènes en toluène et aromatiques à 9 atomes de carbone ;

- transfert d'alkyles entre l'éthylbenzène et les xylènes ; et entre les xylènes entre eux ;

- ouverture de cycles naphténiques et craquage.

L'ensemble de ces réactions engendre la production de molécules moins valorisables, qui ne sont pas recyclées dans la « boucle $C_8$ aromatiques » et sont considérées comme des pertes nettes pour le procédé. Sont ainsi considérées comme pertes nettes toutes les molécules autres que les molécules cycliques à huit atomes de carbone.

Les réactions d'isomérisation ainsi que les réactions parasites sont principalement catalysées par la fonction acide. Les propriétés de la zéolithe (nombre et force des sites acides de Bronsted, topologie du réseau microporeux, etc...), faisant office de fonction acide, ont ainsi un impact direct sur les propriétés du catalyseur bifonctionnel, et notamment sa sélectivité.

La catalyse de l'isomérisation d'une coupe C$_8$ aromatiques en xylènes a ainsi fait l'objet de nombreux brevets portant sur différentes zéolithes. Parmi les zéolithes utilisées en isomérisation d'une coupe C$_8$ aromatiques, on trouve la ZSM-5, utilisée seule ou en mélange avec d'autres zéolithes, comme par exemple la mordénite. Ces catalyseurs sont notamment décrits dans les brevets US 4 467 129 B et US 4 482 773 B. D'autres catalyseurs principalement à base de mordénite ont été décrits par exemple dans le brevet FR 2 477 903 B. Il a également été proposé un catalyseur à base d'une zéolithe de type structural EUO (EP 923 987) ou à base d'une zéolithe de type structural MTW (WO 2005 065380 A, WO 2010 000652 A, US 2014 0296601 A) ou encore à base d'une zéolithe UZM-8 dans le brevet US 7 091 190 B.

Ces exemples illustrent la recherche continue effectuée pour développer des catalyseurs toujours plus performants pour l'isomérisation de coupes C$_8$ aromatiques, notamment en minimisant la production des pertes nettes par la mise en oeuvre de zéolithes appropriées. Pour une structure zéolithique donnée, deux facteurs sont connus pour avoir un impact sur sa sélectivité, et donc sur la sélectivité du catalyseur. Le premier facteur est la densité de sites acides de Brønsted : l'activité par site acide pour les réactions bimoléculaires comme la dismutation de l'éthylbenzène est proportionnelle à la densité de sites acides (M. Guisnet, Techniques de l'Ingénieur, j1217). De ce point de vue il serait donc souhaitable d'utiliser une zéolithe présentant une densité de sites faible pour minimiser les réactions parasites de dismutation et de transalkylation. Le second facteur est la force des sites acides : la réaction de désalkylation de l'éthylbenzène en benzène implique la formation d'ions éthylcarbéniums instables avec une haute barrière énergétique (P. Moreau, thèse à l'université de Poitiers, 2005) et nécessite la présence de sites acides forts. Cette réaction est ainsi très sensible à la force des sites acides de la zéolithe. De ce point de vue il est donc souhaitable d'utiliser une zéolithe présentant peu de sites acides forts pour minimiser ce type de réaction parasite. Or, pour une structure zéolithique donnée, la force des sites acides est fonction de leur densité : les sites acides sont d'autant plus forts que ceux-ci sont isolés, et que donc leur densité est faible (C. Marcilly, catalyse acido-basique, volume 1, 2003). De ce point de vue il serait donc souhaitable d'utiliser une zéolithe présentant une densité de site forte pour minimiser les réactions parasites de désalkylation. La minimisation des différentes réactions parasites requiert donc des exigences contradictoires en termes de densité de sites acides. De plus, *in fine* la densité de sites acides et leur force dictent également l'activité globale de la zéolithe.

A ce titre pour une structure zéolithique donnée il est nécessaire d'identifier la gamme de densité de sites acides optimale permettant d'obtenir le meilleur compromis entre activité et sélectivité. La densité de sites acides d'une zéolithe est entre autre fonction du rapport entre le nombre de moles de silicium et le nombre de moles d'aluminium de ladite zéolithe.

WO 2010/015733 décrit un catalyseur comprenant une zéolithe IZM 2 présentant une composition chimique, par la formule générale suivante : XO$_2$ : aY$_2$O$_3$ dans laquelle X représente au moins un élément tétravalent, Y représente au moins un élément trivalent. La demande enseigne que ledit catalyseur peuvent être mis is en oeuvre dans des procédés de transformation d'hydrocarbure tel que l'isomérisation des composés aromatiques à 8 atomes de carbone (C8), la transalkylation des composés alkylaromatiques, l'hydroisomérisation de paraffines linéaires légères et la transformation d'alcools.

Récemment, la demanderesse dans ses travaux a mis au point une nouvelle zéolithe, la zéolithe IZM-2 (FR 2 918 050 B) incorporée dans la présente demande par référence, ainsi que son utilisation dans un catalyseur d'isomérisation d'une coupe contenant au moins un composé aromatique à huit atomes de carbone (FR 2 934 793 B). FR 2 918 050 B divulgue un solide IZM-2 présentant un rapport molaire compris entre 1 et 500. Dans l'exemple illustratif du brevet FR 2 934 793 B, seul un solide IZM-2 présentant un rapport molaire Si/Al de 53 est utilisé pour l'isomérisation d'une coupe contenant au moins un composé aromatique à huit atomes de carbone.

Aussi, un objectif de la présente invention est de fournir un procédé d'isomérisation de la coupe C$_8$ aromatiques comprenant une zéolithe IZM-2 de rapport molaire Si/Al optimisé, laquelle présente un diagramme de diffraction de rayons X tel que donné ci-dessous, pour limiter la production des pertes nettes.

**Résumé de l'invention**

La présente invention concerne un procédé d'isomérisation d'une coupe C$_8$ aromatiques mettant en œuvre un catalyseur comprenant au moins une zéolithe IZM-2, au moins une matrice et au moins un métal du groupe VIII de la classification périodique des éléments, ledit catalyseur étant caractérisé en ce que le rapport entre le nombre de moles de silicium et le nombre de moles d'aluminium de la zéolithe IZM-2 est compris entre 60 et 95.

Au sens de la présente invention on entend par rapport entre le nombre de moles de silicium et le nombre de moles d'aluminium de la zéolithe IZM-2, le rapport du nombre de moles de silicium divisé par le nombre de moles d'aluminium du réseau de la zéolithe IZM-2.

Le catalyseur est utilisé dans un procédé d'isomérisation d'une coupe aromatique comprenant au moins un composé aromatique à huit atomes de carbone par molécule, dans les conditions opératoires suivantes :

- une température de 300°C à 500°C,

- une pression partielle d'hydrogène de 0,3 à 1,5 MPa,
- une pression totale de 0,45 à 1,9 MPa, et
- une vitesse spatiale d'alimentation, exprimée en kilogramme de charge introduite par kilogramme de catalyseur et par heure, de 0,25 à 30 $h^{-1}$.

Il a été découvert, de façon surprenante que le catalyseur comprenant au moins une matrice, au moins un métal du groupe VIII de la classification périodique des éléments, et au moins la zéolithe IZM-2 dont le rapport molaire Si/Al est compris entre 60 et 95, de manière plus préférée entre 60 et 80 et de manière encore plus préférée entre 60 et 70 conduit à des performances catalytiques améliorées en terme de sélectivité lors d'un procédé d'isomérisation d'une charge aromatique comprenant au moins une coupe aromatique comprenant au moins un composé aromatique à huit atomes de carbone par molécule. Un tel catalyseur est sensiblement plus sélectif qu'un catalyseur comprenant une zéolithe IZM-2 dont le rapport molaire Si/Al est inférieur à 60. Il en résulte ainsi une diminution de la production des pertes nettes pour l'obtention d'un rendement donné en para-xylène lorsque le procédé d'isomérisation est mis en oeuvre en présence du catalyseur

**Description détaillée de l'invention**

La présente invention porte sur un procédé d'isomérisation d'une coupe $C_8$ aromatiques mettant en œuvre un catalyseur comprenant, et de préférence constitué de, au moins une zéolithe IZM-2 contenant des atomes de silicium et des atomes d'aluminium, au moins une matrice et au moins un métal du groupe VIII de la classification périodique des éléments, ledit catalyseur étant caractérisé en ce que le rapport entre le nombre de moles de silicium et le nombre de moles d'aluminium de la zéolithe IZM-2 est compris entre 60 et 95.

Le catalyseur est caractérisé en ce qu'il comprend une zéolithe IZM-2 dont le rapport du nombre de moles de silicium divisé par le nombre de moles d'aluminium du réseau de la zéolithe est entre 60 et 95, de manière plus préférée entre 60 et 80 et de manière encore plus préférée entre 60 et 70.

**Zéolithe IZM-2**

Conformément à l'invention, le catalyseur comprend la zéolithe IZM-2. La zéolithe IZM-2 présente un diagramme de diffraction de rayons X incluant au moins les raies inscrites dans le tableau 1. IZM-2 présente une structure cristalline.

Avantageusement, le diagramme de diffraction est obtenu par analyse radiocristallographique au moyen d'un diffractomètre en utilisant la méthode classique des poudres avec le rayonnement $K_{\alpha 1}$ du cuivre ($\lambda$ = 1,5406 Å). A partir de la position des pics de diffraction représentée par l'angle 2θ, on calcule, par la relation de Bragg, les équidistances réticulaires $d_{hkl}$ caractéristiques de l'échantillon. L'erreur de mesure $\Delta(d_{hkl})$ sur $d_{hkl}$ est calculée grâce à la relation de Bragg en fonction de l'erreur absolue $\Delta(2\theta)$ affectée à la mesure de 2θ. Une erreur absolue $\Delta(2\theta)$ égale à $\pm$ 0,02° est communément admise. L'intensité relative $I_{rel}$ affectée à chaque valeur de $d_{hkl}$ est mesurée d'après la hauteur du pic de diffraction correspondant. Le diagramme de diffraction des rayons X de IZM-2 selon l'invention comporte au moins les raies aux valeurs de $d_{hkl}$ données dans le tableau 1. Dans la colonne des $d_{hkl}$, on indique les valeurs moyennes des distances inter-réticulaires en Angstrôms (Å). Chacune de ces valeurs doit être affectée de l'erreur de mesure $\Delta_{(dhkl)}$ comprise entre $\pm$ 0,6Å et $\pm$ 0,01 Å.

Tableau 1: valeurs moyennes des $d_{hkl}$ et intensités relatives mesurées sur un diagramme de diffraction de rayons X du solide cristallisé IZM-2 calciné.

| 2 thêta (°) | $d_{hkl}$ (Å) | $I_{rel}$ | 2 thêta (°) | $d_{hkl}$ (Å) | $I_{rel}$ |
|---|---|---|---|---|---|
| 5,07 | 17,43 | ff | 19,01 | 4,66 | ff |
| 7,36 | 12,01 | FF | 19,52 | 4,54 | ff |
| 7,67 | 11,52 | FF | 21,29 | 4,17 | m |
| 8,78 | 10,07 | F | 22,44 | 3,96 | f |
| 10,02 | 8,82 | ff | 23,10 | 3,85 | mf |
| 12,13 | 7,29 | ff | 23,57 | 3,77 | f |
| 14,76 | 6,00 | ff | 24,65 | 3,61 | ff |
| 15,31 | 5,78 | ff | 26,78 | 3,33 | f |
| 15,62 | 5,67 | ff | 29,33 | 3,04 | ff |

(suite)

| 2 thêta (°) | $d_{hkl}$ (Å) | $I_{rel}$ | 2 thêta (°) | $d_{hkl}$ (Å) | $I_{rel}$ |
|---|---|---|---|---|---|
| 16,03 | 5,52 | ff | 33,06 | 2,71 | ff |
| 17,60 | 5,03 | ff | 36,82 | 2,44 | ff |
| 18,22 | 4,87 | ff | 44,54 | 2,03 | ff |
| où FF = très fort ; F = fort ; m = moyen ; mf = moyen faible ; f = faible ; ff = très faible. | | | | | |

L'intensité relative $I_{rel}$ est donnée en rapport à une échelle d'intensité relative où il est attribué une valeur de 100 à la raie la plus intense du diagramme de diffraction des rayons X : ff <15 ; 15 ≤f <30 ; 30 ≤ mf <50 ; 50 ≤m < 65 ; 65 ≤F < 85 ; FF ≥ 85.

IZM-2 présente une composition chimique exprimée sur une base anhydre, en termes de moles d'oxydes, définie par la formule générale suivante : $SiO_2$ : a $Al_2O_3$ : b $M_{2/n}O$, dans laquelle M est au moins un métal alcalin et/ou un métal alcalino-terreux de valence n. Dans ladite formule donnée ci-dessus, a représente le nombre de moles de $Al_2O_3$ et b représente le nombre de moles de $M_{2/n}O$.

Conformément à l'invention, le nombre de moles d'aluminium de réseau par gramme de zéolithe IZM-2 se détermine à partir du pourcentage (%) poids (pds) en aluminium de la zéolithe et du pourcentage d'aluminium tétracoordinés et pentacoordinés présent dans la zéolithe selon la formule :

$$n_{Al} = [(\%pdsAl) * (\%RMNAl^{IV} + \%RMNAl^{V})] / [MM(Al) * 10000]$$

avec

n$_{Al}$: moles d'aluminium de réseau par gramme de zéolithe, en mole/gramme,
%pdsAl: pourcentage poids d'aluminium dans la zéolithe (masse sèche), mesuré par plasma à couplage inductif (ICP) sur un appareil SPECTRO ARCOS ICP-OES de SPECTRO selon la méthode ASTM D7260,
MM(Al): masse molaire de l'aluminium, en gramme/mole,
%RMNAl$^{IV}$: pourcentage pondéral d'aluminium tétracoordiné dans la zéolithe, mesuré par résonance magnétique nucléaire de $^{27}$Al,
%RMNAl$^{V}$: pourcentage pondéral d'aluminium pentacoordiné dans la zéolithe, mesuré par résonance magnétique nucléaire de $^{27}$Al.

Le pourcentage pondéral des atomes d'aluminium tétracoordinés et pentacoordinés présents dans la zéolithe IZM-2 est déterminé par résonance magnétique nucléaire du solide de $^{27}$Al. La RMN de l'aluminium est en effet connue pour être utilisée en vue de repérer et de quantifier les différents états de coordination de ce noyau ("Analyse physico-chimiques des catalyseurs industriels", J. Lynch, Editions Technip (2001) chap. 13, pages 290 et 291). Le spectre RMN de l'aluminium de la zéolithe IZM-2 peut présenter trois signaux, un premier étant caractéristique de la résonance des atomes d'aluminium tétracoordinés, un second étant caractéristique des atomes d'aluminium pentacoordinés et un troisième étant caractéristique de la résonance des atomes d'aluminium hexacoordinés. Les atomes d'aluminium tétracoordinés (noté Al$^{IV}$) résonnent à un déplacement chimique typiquement compris entre +50 ppm et +70 ppm, les atomes d'aluminium pentacoordinés (noté Al$^{V}$) résonnent à un déplacement chimique typiquement compris entre +20 ppm et +40 ppm et les atomes d'aluminium hexacoordinés (noté Al$^{VI}$) résonnent à un déplacement chimique typiquement compris entre -20 ppm et +10 ppm. Le pourcentage pondéral des différentes espèces aluminiques est quantifié à partir de l'intégration des signaux correspondant à chacune de ces espèces. Plus précisément, la zéolithe IZM-2 présente dans le catalyseur selon l'invention a été analysée par RMN-MAS du solide $^{27}$Al sur un spectromètre Brücker de type Avance 400 MHz à l'aide d'une sonde 4 mm optimisée pour $^{27}$Al. La vitesse de rotation de l'échantillon est voisine de 12 kHz. L'atome d'aluminium est un noyau quadripolaire dont le spin est égal à 5/2. Dans des conditions d'analyse dites sélectives, à savoir un champ de radiofréquence faible égal à 30 kHz, un angle d'impulsion faible égal à $\pi/2$ et en présence d'un échantillon saturé en eau, la technique de RMN de rotation à l'angle magique (MAS), notée RMN-MAS, est une technique quantitative. La décomposition de chaque spectre RMN-MAS permet d'accéder directement à la quantité des différentes espèces aluminiques, à savoir des atomes d'aluminium tétracoordinés Al$^{IV}$, pentacoordinés Al$^{V}$ et hexacoordinés Al$^{VI}$. Chaque spectre est calé en déplacement chimique par rapport à une solution molaire de nitrate d'aluminium pour laquelle le signal d'aluminium est à zéro ppm. Les signaux caractérisant les atomes d'aluminium tétracoordinés Al$^{IV}$ sont intégrés typiquement entre +50 ppm et +70 ppm ce qui correspond à l'aire 1, les signaux caractérisant les atomes d'aluminium pentacoordinés Al$^{V}$ sont intégrés typiquement entre +20 ppm et +40 ppm ce qui correspond à l'aire 2 et les signaux caractérisant les atomes d'aluminium hexacoordinés $Al^{VI}$ sont intégrés typiquement entre -20 ppm et +10 ppm ce qui correspond à l'aire 3. Le pourcentage pondéral de chaque espèce aluminique est calculé à partir du rapport entre son aire et l'aire totale. Par exemple le pourcentage pondéral d'atomes d'aluminium hexacoordinés $Al^{VI}$ (noté $\%RMNAl^{VI}$) est calculé comme suit :

$$\%RMNAl^{VI} = (\text{aire } 3) * 100 / (\text{aire } 1 + \text{aire } 2 + \text{aire } 3)$$

Avantageusement, la zéolithe IZM-2 selon l'invention présente un pourcentage pondéral d'atomes d'aluminium hexacoordinés $Al^{VI}$ (noté $\%RMNAl^{VI}$) inférieur à 50%, de manière préférée inférieur à 40%, et de manière très préférée inférieur à 30%.

Le nombre de moles de silicium par gramme de zéolithe IZM-2 se détermine à partir du pourcentage (%) poids (pds) en silicium de la zéolithe selon la formule :

$$n_{Si} = [(\%pdsSi) / [MM(Si) * 100]$$

avec

$n_{Si}$ : moles de silicium par gramme de zéolithe, en mole/gramme,
%pdsSi : pourcentage poids d'aluminium dans la zéolithe (masse sèche), mesuré par Fluorescence X par dosage en perle sur un appareil AXIOS de marque PANalytical travaillant à 125 mA and 32 kV,
MM(Si) : masse molaire du silicium, en gramme/mole.

Le rapport du nombre de moles de silicium divisé par le nombre de moles d'aluminium de réseau de la zéolithe IZM-2 (Si/Al) est calculé selon la formule :

$$Si/Al = n_{Si} / n_{Al}$$

avec

$n_{Si} / n_{Al}$ : rapport du nombre de moles de silicium divisé par le nombre de moles d'aluminium de réseau, en mole/mole,
$n_{Si}$ : moles de silicium par gramme de la zéolithe, en mole/gramme,
$n_{Al}$ : moles d'aluminium de réseau par gramme de zéolithe, en mole/gramme.

La zéolithe IZM-2, comprise dans le catalyseur selon l'invention présente un rapport molaire Si/Al compris entre 60 et 95, de manière plus préférée entre 60 et 80 et de manière encore plus préférée entre 60 et 70.

Conformément à l'invention, le rapport molaire Si/Al désiré pour la zéolithe IZM-2 peut être obtenu directement lors de l'étape de synthèse de la zéolithe par ajustement des conditions de synthèses et notamment de la composition du gel de synthèse en contrôlant par exemple les quantités relatives de silicium et d'aluminium engagées dans le gel de synthèse.

Un procédé de préparation de la zéolithe IZM-2 est enseigné dans le brevet FR 2 918 050 B incorporé ici par référence.

De manière avantageuse, on fait réagir un mélange aqueux comportant au moins une source d'au moins un oxyde $SiO_2$, éventuellement au moins une source d'au moins un oxyde $Al_2O_3$, éventuellement au moins une source d'au moins un métal alcalin et/ou alcalino-terreux de valence n, et de préférence au moins une espèce organique R comportant deux atomes d'azote quaternaires, le mélange présentant préférentiellement la composition molaire suivante :

$SiO_2/Al_2O_3$: au moins 2, de préférence au moins 20, de manière plus préférée de 60 à 600, $H_2O/SiO_2$: 1 à 100, de préférence de 10 à 70,

$R/SiO_2$: 0,02 à 2, de préférence de 0,05 à 0,5,

$M_{2/n}O/SiO_2$: 0 à 1, de préférence de 0,005 et 0,5,

où M est un ou plusieurs métal(aux) alcalin(s) et/ou alcalino-terreux choisi(s) parmi le lithium, le sodium, le potassium, le calcium, le magnésium et le mélange d'au moins deux de ces métaux, de préférence M est le sodium. Avantageusement, l'élément R est le 1,6-bis(méthylpiperidinium)hexane.

Conformément à l'invention, le rapport molaire Si/Al de la zéolithe IZM-2 peut être aussi ajusté à la valeur désirée par des méthodes de post traitement de la zéolithe IZM-2 obtenue après synthèse. De telles méthodes sont connues de l'homme du métier, et permettent d'effectuer de la désalumination ou de la désilication de la zéolithe. De manière préférée le rapport molaire Si/Al de la zéolithe IZM-2 entrant dans la composition du catalyseur selon l'invention est ajusté par un choix approprié des conditions de synthèse de ladite zéolithe.

La zéolithe IZM-2 présente dans le catalyseur selon l'invention, se trouve très avantageusement sous sa forme acide c'est-à-dire sous forme protonée $H^+$. Dans un tel cas, il est avantageux que le rapport du nombre de moles de cation autre que le proton par gramme de zéolithe IZM-2 divisé par le nombre de moles d'aluminium de réseau par gramme de zéolithe IZM-2 soit inférieur à 0,9, de préférence inférieur à 0,6 et de manière très préférée inférieur à 0,3. Pour ce faire, la zéolithe IZM-2 entrant dans la composition du catalyseur selon l'invention peut être par exemple échangée par au moins un traitement par une solution d'au moins un sel d'ammonium de manière à obtenir la forme ammonium de la zéolithe IZM-2 qui une fois calcinée conduit à la forme acide de ladite zéolithe IZM-2. Cette étape d'échange peut être effectuée à toute étape de la préparation du catalyseur, c'est-à-dire après l'étape de préparation de la zéolithe IZM-2, après l'étape de mise en forme de la zéolithe IZM-2 avec une matrice, ou encore après l'étape d'introduction du métal hydro-déshydrogénant. De préférence l'étape d'échange est effectuée après l'étape de mise en forme de la zéolithe IZM-2.

## Matrice

Conformément à l'invention, le catalyseur comprend au moins une matrice. Ladite matrice peut avantageusement être amorphe ou cristallisée.

De préférence, ladite matrice est avantageusement choisie dans le groupe formé par l'alumine, la silice, la silice-alumine, les argiles, l'oxyde de titane, l'oxyde de bore et la zircone, pris seuls ou en mélange ou bien on peut choisir également les aluminates. De préférence, l'alumine est utilisée comme matrice. De manière préférée, ladite matrice contient de l'alumine sous toutes ses formes connues de l'homme du métier, telles que par exemple les alumines de type alpha, gamma, êta, delta. Lesdites alumines diffèrent par leur surface spécifique et leur volume poreux. Le mélange de la matrice et de la zéolithe IZM-2 mis en forme constitue le support du catalyseur.

## Phase métallique

Conformément à l'invention, le catalyseur comprend au moins un métal du groupe VIII de préférence choisi parmi le fer, le cobalt, le nickel, le ruthénium, le rhodium, le palladium, l'osmium, l'iridium et le platine, de préférence choisi parmi les métaux nobles du groupe VIII, de manière très préférée choisi parmi le palladium et le platine et de manière encore plus préférée on choisit le platine.

La dispersion du(es) métal(ux) du groupe VIII, déterminée par chimisorption, par exemple par titration $H_2/O_2$ ou par chimisorption du monoxyde de carbone, est comprise entre 10% et 100%, de préférence entre 20% et 100% et de manière encore plus préférée entre 30% et 100%. Le coefficient de répartition macroscopique du(es) métal(ux) du groupe VIII, obtenu à partir de son (leur) profil déterminé par microsonde de Castaing, défini comme le rapport des concentrations du(es) métal(ux) du groupe VIII au cœur du grain par rapport au bord de ce même grain, est compris entre 0,7 et 1,3, de préférence entre 0,8 et 1,2. La valeur de ce rapport, voisine de 1, témoigne de l'homogénéité de la répartition du(es) métal(ux) du groupe VIII dans le catalyseur.

Ledit catalyseur peut comprendre avantageusement au moins un métal additionnel choisi dans le groupe formé par les métaux des groupes IIIA, IVA et VIIB de la classification périodique des éléments et de préférence choisi parmi le gallium, l'indium, l'étain et le rhénium. Ledit métal additionnel est de préférence choisi parmi l'indium, l'étain et le rhénium.

## Préparation du catalyseur

Le catalyseur selon l'invention peut avantageusement être préparé selon toutes les méthodes bien connues de l'homme du métier.

## Mise en forme

Avantageusement, les différents constituants du support ou du catalyseur peuvent être mis en forme par étape de malaxage pour former une pâte puis extrusion de la pâte obtenue, ou alors par mélange de poudres puis pastillage, ou alors par tout autre procédé connu d'agglomération d'une poudre contenant de l'alumine. Les supports ainsi obtenus peuvent se présenter sous différentes formes et dimensions. De manière préférée la mise en forme est effectuée par malaxage et extrusion.

Lors de la mise en forme du support par malaxage puis extrusion, ladite zéolithe IZM-2 peut être introduite au cours de la mise en solution ou en suspension des composés d'alumine ou précurseurs d'alumine tels que la boéhmite par exemple. Ladite zéolithe IZM-2 peut être, sans que cela soit limitatif, par exemple sous forme de poudre, poudre broyée, suspension, suspension ayant subi un traitement de désagglomération. Ainsi, par exemple, ladite zéolithe peut avantageusement être mise en suspension acidulée ou non à une concentration ajustée à la teneur finale en IZM-2 visée dans le catalyseur selon l'invention. Cette suspension appelée couramment une barbotine est alors mélangée avec les composés d'alumine ou précurseurs d'alumine.

Par ailleurs, l'utilisation d'additifs peut avantageusement être mise en oeuvre pour faciliter la mise en forme et/ou améliorer les propriétés mécaniques finales des supports comme cela est bien connu par l'homme du métier. A titre d'exemple d'additifs, on peut citer notamment la cellulose, la carboxyméthyl-cellulose, la carboxy-éthyl-cellulose, du tall-oil (huile de tall), les gommes xanthaniques, des agents tensio-actifs, des agents floculants comme les polyacrylamides, le noir de carbone, les amidons, l'acide stéarique, l'alcool polyacrylique, l'alcool polyvinylique, des biopolymères, le glucose, les polyéthylènes glycols, etc.

On peut avantageusement ajouter ou retirer de l'eau pour ajuster la viscosité de la pâte à extruder. Cette étape peut avantageusement être réalisée à tout stade de l'étape de malaxage.

Pour ajuster la teneur en matière solide de la pâte à extruder afin de la rendre extrudable, on peut également ajouter un composé majoritairement solide et de préférence un oxyde ou un hydrate. On utilise de manière préférée un hydrate et de manière encore plus préférée un hydrate d'aluminium. La perte au feu de cet hydrate est avantageusement supérieure à 15%.

L'extrusion de la pâte issue de l'étape de malaxage peut avantageusement être réalisée par n'importe quel outil conventionnel, disponible commercialement. La pâte issue du malaxage est avantageusement extrudée à travers une filière, par exemple à l'aide d'un piston ou d'une mono-vis ou double vis d'extrusion. L'extrusion peut avantageusement être réalisée par toute méthode connue de l'homme de métier.

Les supports du catalyseur selon l'invention sont en général sous la forme d'extrudés cylindriques ou polylobés tels que bilobés, trilobés, polylobés de forme droite ou torsadée, mais peuvent éventuellement être fabriqués et employés sous la forme de poudres concassées, de tablettes, d'anneaux, de billes et/ou de roues. De préférence, les supports du catalyseur selon l'invention ont la forme de sphères ou d'extrudés. Avantageusement le support se présente sous forme d'extrudés d'un diamètre compris entre 0,5 et 5 mm et plus particulièrement entre 0,7 et 2,5 mm. Les formes peuvent être cylindriques (qui peuvent être creuses ou non) et/ou cylindriques torsadés et/ou multilobées (2, 3, 4 ou 5 lobes par exemple) et/ou anneaux. La forme multilobée est avantageusement utilisée de manière préférée.

Séchage

Le support ainsi obtenu peut ensuite être soumis à une étape de séchage. Ladite étape de séchage est avantageusement effectuée par toute technique connue de l'homme du métier.

De préférence, le séchage est effectué sous flux d'air. Ledit séchage peut également être effectué sous flux de tout gaz oxydant, réducteur ou inerte. De préférence, le séchage est avantageusement effectué à une température comprise entre 50 et 180°C, de manière préférée entre 60 et 150°C et de manière très préférée entre 80 et 130°C.

Calcination

Ledit support, éventuellement séché, subit ensuite de préférence une étape de calcination.

Ladite étape de calcination est avantageusement réalisée en présence d'oxygène moléculaire, par exemple en effectuant un balayage d'air, à une température avantageusement supérieure à 200°C et inférieure ou égale à 1100°C. Ladite étape de calcination peut avantageusement être effectuée en lit traversé, en lit léché ou en atmosphère statique. Par exemple, le four utilisé peut être un four rotatif tournant ou être un four vertical à couches traversées radiales. De préférence, ladite étape de calcination est effectuée entre plus d'une heure à 200°C à moins d'une heure à 1100°C. La calcination peut avantageusement être opérée en présence de vapeur d'eau et/ou en présence d'une vapeur acide ou basique. Par exemple, la calcination peut être réalisée sous pression partielle d'ammoniaque.

Traitements post-calcination

Des traitements post-calcination peuvent éventuellement être effectués, de manière à améliorer les propriétés du support, notamment les propriétés texturales.

Ainsi, le support du catalyseur mis en œuvre dans le procédé selon la présente invention peut être soumis à un traitement hydrothermal en atmosphère confinée. On entend par traitement hydrothermal en atmosphère confinée un traitement par passage à l'autoclave en présence d'eau à une température supérieure à la température ambiante, de préférence supérieure à 25°C, de préférence supérieure à 30°C.

Au cours de ce traitement hydrothermal, on peut avantageusement imprégner le support, préalablement à son passage à l'autoclave (l'autoclavage étant fait soit en phase vapeur, soit en phase liquide, cette phase vapeur ou liquide de l'autoclave pouvant être acide ou non). Cette imprégnation, préalable à l'autoclavage, peut avantageusement être acide ou non. Cette imprégnation, préalable à l'autoclavage peut avantageusement être effectuée à sec ou par immersion du support dans une solution aqueuse acide. Par imprégnation à sec, on entend mise en contact du support avec un volume de solution inférieur ou égal au volume poreux total du support. De préférence, l'imprégnation est réalisée à sec. L'autoclave est de préférence un autoclave à panier rotatif tel que celui défini dans la demande brevet EP 0 387 109 A. La température pendant l'autoclavage peut être comprise entre 100 et 250°C pendant une période de temps comprise entre 30 minutes et 3 heures.

Dépôt de la phase métallique

Pour le dépôt du métal du groupe VIII de la classification périodique des éléments, toutes les techniques de dépôt connues de l'homme du métier et tous les précurseurs de tels métaux peuvent convenir. On peut utiliser des techniques de dépôt par imprégnation à sec ou en excès d'une solution contenant les précurseurs des métaux, en présence de compétiteurs ou non. L'introduction du métal peut s'effectuer à toute étape de la préparation du catalyseur: sur la zéolithe IZM-2 et/ou sur la matrice, notamment avant l'étape de mise en forme, pendant l'étape de mise en forme, ou après l'étape de mise en forme, sur le support du catalyseur. De manière préférée le dépôt du métal s'effectue après l'étape de mise en forme.

Le contrôle de certains paramètres mis en oeuvre lors du dépôt, en particulier la nature du précurseur du (des) métal(ux) du groupe VIII utilisé(s), permet d'orienter le dépôt du(es)dit(s) métal(ux) majoritairement sur la matrice ou sur la zéolithe.

Ainsi, pour introduire le(s) métal(ux) du groupe VIII, préférentiellement le platine et/ou le palladium, majoritairement sur la matrice, on peut mettre en oeuvre un échange anionique avec de l'acide hexachloroplatinique et/ou de l'acide hexachloropalladique, en présence d'un agent compétiteur, par exemple de l'acide chlorhydrique, le dépôt étant en général suivi d'une calcination, par exemple à une température comprise entre 350 et 550°C et pendant une durée comprise entre 1 et 4 heures. Avec de tels précurseurs, le(s) métal(ux) du groupe VIII est(sont) déposé(s) majoritairement sur la matrice et le(s)dit(s) métal(ux) présente(nt) une bonne dispersion et une bonne répartition macroscopique à travers le grain de catalyseur.

On peut aussi envisager de déposer le(s) métal(ux) du groupe VIII, préférentiellement le platine et/ou le palladium, par échange cationique de manière à ce que le(s)dit(s) métal(ux) soi(en)t déposé(s) majoritairement sur la zéolithe. Ainsi, dans le cas du platine, le précurseur peut être par exemple choisi parmi :

- les composés ammoniaqués tels que les sels de platine (II) tétramines de formule $Pt(NH_3)_4X_2$, les sels de platine (IV) hexamines de formule $Pt(NH_3)_6X_4$ ; les sels de platine (IV) halogénopentamines de formule $(PtX(NH_3)_5)X_3$ ; les sels de platine N-tétrahalogénodiamines de formule $PtX_4(NH_3)_2$ ; et

- les composés halogénés de formule $H(Pt(acac)_2X)$;

X étant un halogène choisi dans le groupe formé par le chlore, le fluor, le brome et l'iode, X étant de préférence le chlore, et "acac" représentant le groupe acétylacétonate (de formule brute $C_5H_7O_2$), dérivé de l'acétylacétone. Avec de tels précurseurs, le(s) métal(ux) du groupe VIII est(sont) déposé(s) majoritairement sur la zéolithe et le(s)dit(s) métal(ux) présente(nt) une bonne dispersion et une bonne répartition macroscopique à travers le grain de catalyseur.

Dans le cas où le catalyseur de l'invention contient également au moins un métal choisi parmi les métaux des groupes IIIA, IVA et VIIB, toutes les techniques de dépôt d'un tel métal connues de l'homme du métier et tous les précurseurs de tels métaux peuvent convenir.

On peut ajouter le(s) métal(ux) du groupe VIII et celui(ceux) des groupes IIIA, IVA et VIIB, soit séparément soit simultanément dans au moins une étape unitaire. Lorsqu'au moins un métal des groupes IIIA, IVA et VIIB est ajouté séparément, il est préférable qu'il soit ajouté après le métal du groupe VIII.

Le métal additionnel choisi parmi les métaux des groupes IIIA, IVA et VIIB peut être introduit par l'intermédiaire de composés tels que par exemple les chlorures, les bromures et les nitrates des métaux des groupes IIIA, IVA et VIIB. Par exemple dans le cas de l'indium, on utilise avantageusement le nitrate ou le chlorure et dans le cas du rhénium, on utilise avantageusement l'acide perrhénique. Le métal additionnel choisi parmi les métaux des groupes IIIA, IVA et VIIB peut également être introduit sous la forme d'au moins un composé organique choisi dans le groupe constitué par les complexes dudit métal, en particulier les complexes polycétoniques du métal et les hydrocarbylmétaux tels que les alkyles, les cycloalkyles, les aryles, les alkylaryles et les arylalkyles de métaux. Dans ce dernier cas, l'introduction du métal est avantageusement effectuée à l'aide d'une solution du composé organométallique dudit métal dans un solvant organique. On peut également employer des composés organohalogénés du métal. Comme composés organiques de métaux, on peut citer en particulier le tétrabutylétain, dans le cas de l'étain, et le triphénylindium, dans le cas de l'indium.

Si le métal additionnel choisi parmi les métaux des groupes IIIA, IVA et VIIB est introduit avant le métal du groupe VIII, le composé du métal IIIA, IVA et/ou VIIB utilisé est généralement choisi dans le groupe constitué par l'halogénure, le nitrate, l'acétate, le tartrate, le carbonate et l'oxalate du métal. L'introduction est alors avantageusement effectuée en solution aqueuse. Mais il peut également être introduit à l'aide d'une solution d'un composé organométallique du métal par exemple le tétrabutylétain. Dans ce cas, avant de procéder à l'introduction d'au moins un métal du groupe VIII, on procédera à une calcination sous air.

De plus, des traitements intermédiaires tels que par exemple une calcination et/ou une réduction peuvent être appliqués entre les dépôts successifs des différents métaux.

Avant son utilisation dans le procédé selon l'invention, le catalyseur est de préférence réduit. Cette étape de réduction est avantageusement réalisée par un traitement sous hydrogène à une température comprise entre 150°C et 650°C et une pression totale comprise entre 0,1 et 25 MPa. Par exemple, une réduction consiste en un palier à 150°C de deux heures puis une montée en température jusqu'à 450°C à la vitesse de 1°C/min puis un palier de deux heures à 450°C; durant toute cette étape de réduction, le débit d'hydrogène est de 1000 normaux $m^3$ d'hydrogène par tonne catalyseur et la pression totale maintenue constante à 0,2 MPa. Toute méthode de réduction *ex-situ* peut avantageusement être envisagée. Une réduction préalable du catalyseur final *ex situ,* sous courant d'hydrogène, peut être mise en oeuvre, par exemple à une température de 450°C à 600°C, pendant une durée de 0,5 à 4 heures.

Ledit catalyseur comprend également avantageusement du soufre. Dans le cas où le catalyseur de l'invention contient du soufre, celui-ci peut être introduit à n'importe quelle étape de la préparation du catalyseur: avant ou après étape de mise en forme, et/ou séchage et/ou calcination, avant et/ou après l'introduction du ou des métaux cités précédemment, ou encore par sulfuration *in situ* et ou *ex situ* avant la réaction catalytique. Dans le cas d'une sulfuration *in situ,* la réduction, si le catalyseur n'a pas été préalablement réduit, intervient avant la sulfuration. Dans le cas d'une sulfuration *ex situ,* on effectue également la réduction puis la sulfuration. La sulfuration s'effectue de préférence en présence d'hydrogène en utilisant tout agent sulfurant bien connu de l'homme de métier, tel que par exemple le sulfure de diméthyle ou le sulfure d'hydrogène.

Les catalyseurs selon l'invention se présentent sous différentes formes et dimensions. Ils sont utilisés en général sous la forme d'extrudés cylindriques et/ou polylobés tels que bilobés, trilobés, polylobés de forme droite et/ou torsadée, mais peuvent éventuellement être fabriqués et employés sous la forme de poudres concassées, de tablettes, d'anneaux, de billes et/ou de roues. De préférence, les catalyseurs mis en oeuvre dans le procédé selon l'invention ont la forme de sphères ou d'extrudés. Avantageusement le catalyseur se présente sous forme d'extrudés d'un diamètre compris entre 0,5 et 5 mm et plus particulièrement entre 0,7 et 2,5 mm. Les formes peuvent être cylindriques (qui peuvent être creuses ou non) et/ou cylindriques torsadés et/ou multilobées (2, 3, 4 ou 5 lobes par exemple) et/ou anneaux. La forme multilobée est avantageusement utilisée de manière préférée. Le dépôt du métal ne change pas la forme du support.

Ledit catalyseur selon l'invention comprend plus particulièrement, et de préférence est constitué de:

- de 1 à 90%, de préférence de 3 à 80% et de manière encore plus préférée de 4 à 60% poids de la zéolithe IZM-2 selon l'invention,
- de 0,01 à 4%, de préférence de 0,05 à 2% poids d'au moins un métal du groupe VIII de la classification périodique des éléments, de préférence le platine,
- éventuellement de 0,01 à 2%, de préférence de 0,05 à 1% poids d'au moins un métal additionnel choisi dans le groupe formé par les métaux des groupes IIIA, IVA et VIIB,
- éventuellement une teneur en soufre, de préférence telle que le rapport du nombre de moles de soufre sur le nombre de moles de(s) métal(ux) du groupe VIII soit compris entre 0,3 et 3,
- au moins une matrice, de préférence l'alumine, assurant le complément à 100% dans le catalyseur.

**Le procédé d'isomérisation**

La présente invention a pour objet un procédé d'isomérisation d'une coupe contenant au moins un composé aromatique à huit atomes de carbone par molécule, ledit procédé comprenant la mise en contact de ladite coupe aromatique avec au moins ledit catalyseur selon l'invention présent dans un réacteur catalytique.

Ladite coupe aromatique contenant au moins un composé aromatique ayant huit atomes de carbone par molécule comprend en particulier comme composé aromatique ayant huit atomes de carbone par molécule soit uniquement un mélange de xylènes, soit uniquement de l'éthylbenzène, soit un mélange de xylène(s) et d'éthylbenzène.

Ledit procédé d'isomérisation est mis en œuvre généralement selon les conditions opératoires suivantes :

- une température de 300°C à 500°C, de préférence de 320°C à 450°C et de manière encore plus préférée de 340°C à 430°C ;

- une pression partielle d'hydrogène de 0,3 à 1,5 MPa, de préférence de 0,4 et 1,2 MPa et de manière encore préférée de 0,7 à 1,2 MPa ;

- une pression totale de 0,45 à 1,9 MPa, de préférence de 0,6 à 1,5 MPa ; et

- une vitesse spatiale d'alimentation, exprimée en kilogramme de charge introduite par kilogramme de catalyseur et par heure, de 0,25 à 30 $h^{-1}$, de préférence de 1 à 10 $h^{-1}$ et de manière encore préférée de 2 à 6 $h^{-1}$.

Les exemples suivants illustrent l'invention sans toutefois en limiter la portée.

**Exemples**

Exemple 1 (non conforme à l'invention) : préparation du catalyseur d'isomérisation A

Le catalyseur A est un catalyseur comprenant une zéolithe IZM-2, du platine, et une matrice alumine. Cette zéolithe IZM-2 a été synthétisée conformément à l'enseignement du brevet FR 2 918 050 B. Une suspension colloïdale de silice connue sous le terme commercial Ludox HS-40 commercialisée par Aldrich, est incorporée dans une solution composée de soude (Prolabo), de structurant dibromure de 1,6bis(méthylpiperidinium)hexane, d'hydroxyde d'aluminium (Aldrich) et d'eau déionisée. La composition molaire du mélange est la suivante: 1 $SiO_2$; 0,0033 $Al_2O_3$; 0,1666 $Na_2O$; 0,1666 1,6bis(méthylpiperidinium)hexane; 33,3333 $H_2O$. Le mélange est agité vigoureusement pendant une demi-heure. Le mélange est ensuite transféré, après homogénéisation, dans un autoclave de type PARR. L'autoclave est chauffé pendant 5 jours à 170°C sous agitation en tourne broche (30 tours/min). Le produit obtenu est filtré, lavé à l'eau déionisée pour atteindre un pH neutre puis séché une nuit à 100°C en étuve. Le solide est ensuite introduit dans un four à moufle pour y être calciné afin d'éliminer le structurant. Le cycle de calcination comprend une montée en température jusqu'à 200°C, un palier à cette température de deux heures, une montée en température jusqu'à 550°C suivi d'un palier de huit heures à cette température et enfin un retour à température ambiante. Les montées en température sont effectuées avec une rampe de 2°C/min. Le solide ainsi obtenu est ensuite mis sous reflux durant 2 heures dans une solution aqueuse de nitrate d'ammonium (10 ml de solution par gramme de solide, concentration en nitrate d'ammonium de 3 M) afin d'échanger les cations alcalins sodium par des ions ammonium. Cette étape de mise sous reflux est effectuée quatre fois avec une solution fraiche de nitrate d'ammonium, puis le solide est filtré, lavé à l'eau déionisée et séché en étuve une nuit à 100°C. Enfin, pour obtenir la zéolithe sous sa forme acide (protonée $H^+$) on réalise une étape de calcination à 550°C durant dix heures (rampe de montée en température de 2°C/min) en lit traversé sous air sec (2 normaux litres par heure et par gramme de solide). Le solide ainsi obtenu a été analysé par Diffraction des Rayons X et identifié comme étant constitué par de la zéolithe IZM-2. La zéolithe, nommée IZM-2_A, est utilisée pour la préparation du catalyseur. Des caractérisations au moyen des méthodes RMN de l'$^{27}$Al, fluorescence X et ICP permettent d'accéder aux résultats suivants pour l'IZM-2_A :

- pourcentage pondéral d'atomes d'aluminium hexacoordinés $Al^{VI}$ : 9%,

- rapport du nombre de moles de silicium divisé par le nombre de moles d'aluminium de réseau, en mole/mole, Si/Al : 113,

- rapport du nombre de moles de sodium divisé par le nombre de moles d'aluminium de réseau, en mole/mole, Na/Al : 0,22.

Le platine est déposé sur une matrice alumine gamma par une méthode d'imprégnation en excès. L'alumine gamma fournie par la société Axens (GOD200) se présente sous la forme de billes. Tout d'abord on sature les billes par remplissage du volume poreux avec de l'eau distillée (ajout goutte à goutte) puis mouillage durant typiquement 30 minutes. On acidifie ensuite l'alumine avec une solution aqueuse d'acide à hauteur de 5% en poids en chlore par rapport à la masse d'alumine sèche (4 ml de solution par gramme de solide) et on laisse sous agitation durant une heure sur table d'agitation puis la solution est soutirée. On effectue ensuite une imprégnation en excès avec une solution aqueuse d'acide hexachloroplatinique à hauteur de 2% poids de platine par rapport à la masse d'alumine sèche (4 ml de solution par gramme de solide) puis on laisse sous agitation sur table d'agitation durant 24 heures puis on soutire la solution et on rince le solide avec deux fois le volume d'échange d'eau distillée. Le solide est ensuite séché une nuit en étuve à 110°C puis calciné sous débit d'air sec en lit traversé (1 normal litre par heure et par gramme de solide) dans les conditions suivantes:

- montée de la température de l'ambiante à 150°C à 5°C/min,

- palier d'une heure à 150°C,

- montée de 150 à 250°C à 5°C/min,

- palier d'une heure à 250°C,

- montée de 250 à 350°C à 5°C/min,

- palier d'une heure à 350°C,

- montée de 350 à 520°C à 5°C/min,

- palier de deux heures à 520°C,

- descente à l'ambiante.

Des caractérisations par fluorescence X, microsonde de Castaing et titrage $H_2/O_2$ permettent d'accéder aux résultats suivants pour le solide :

- pourcentage en platine (masse sèche) : 1,7%,

- dispersion du platine : 82%,

- coefficient de répartition du platine : 0,99.

Le catalyseur A est alors préparé en combinant la zéolithe IZM-2_A et l'alumine imprégnée de platine de la manière suivante. Tout d'abord on s'assure de la maitrise de la granulométrie des poudres de départ par tamisage de l'IZM-2_A et broyage-tamisage des billes d'alumine imprégnées de platine pour obtenir une granulométrie avantageusement inférieure à 63 microns pour les deux solides. Après pesée des masses de solides désirées, le mélange mécanique (2 grammes masse sèche) des deux poudres est effectué à l'aide d'un broyeur à godet durant 2 minutes, avec une boule et une fréquence de 30 Hz. Le mélange est ensuite pastillé à 4 ton-metric avec une presse hydraulique Carver puis broyé et tamisé à la granulométrie préférentielle de 250-500 microns. La composition du catalyseur A est la suivante :

- 10% poids de la zéolithe IZM-2_A,

- 1,5% poids de platine,

- 88,5% poids d'alumine imprégnée.

Exemple 2 (conforme à l'invention) : préparation du catalyseur d'isomérisation B

Le catalyseur B est un catalyseur comprenant une zéolithe IZM-2, du platine, et une matrice alumine. Cette zéolithe IZM-2 a été synthétisée conformément à l'enseignement du brevet FR 2 918 050 B. Une suspension colloïdale de silice connue sous le terme commercial Ludox HS-40 commercialisée par Aldrich, est incorporée dans une solution composée de soude (Prolabo), de structurant dibromure de 1,6bis(méthylpiperidinium)hexane, d'hydroxyde d'aluminium (Aldrich) et d'eau déionisée. La composition molaire du mélange est la suivante: 1 $SiO_2$, 0,0045 $Al_2O_3$ ; 0,1666 $Na_2O$ ; 0,1666 1,6bis(méthylpiperidinium)hexane; 33,3333 $H_2O$. Le mélange est agité vigoureusement pendant une demi-heure. Le mélange est ensuite transféré, après homogénéisation, dans un autoclave de type PARR. L'autoclave est chauffé pendant 5 jours à 170°C sous agitation en tourne broche (30 tours/min). Le produit obtenu est filtré, lavé à l'eau déionisée pour atteindre un pH neutre puis séché une nuit à 100°C en étuve. Le solide est ensuite introduit dans un four à moufle pour y être calciné afin d'éliminer le structurant. Le cycle de calcination comprend une montée en température jusqu'à 200°C, un palier à cette température de deux heures, une montée en température jusqu'à 550°C suivi d'un palier de huit heures à cette température et enfin un retour à température ambiante. Les montées en température sont effectuées avec une rampe de 2°C/min. Le solide ainsi obtenu est ensuite mis sous reflux durant 2 heures dans une solution aqueuse de nitrate d'ammonium (10 ml de solution par gramme de solide, concentration en nitrate d'ammonium de 3 M) afin d'échanger les cations alcalins sodium par des ions ammonium. Cette étape de mise sous reflux est effectuée quatre fois avec une solution fraiche de nitrate d'ammonium, puis le solide est filtré, lavé à l'eau déionisée et séché en étuve une nuit à 100°C. Enfin, pour obtenir la zéolithe sous sa forme acide (protonée H$^+$) on réalise une étape de calcination à 550°C durant dix heures (rampe de montée en température de 2°C/min) en lit traversé sous air sec (2 normaux litres par heure et par gramme de solide). Le solide ainsi obtenu a été analysé par Diffraction des Rayons X et identifié comme étant constitué par de la zéolithe IZM-2. La zéolithe, nommée IZM-2_B, est utilisée pour la préparation du catalyseur. Des caractérisations au moyen des méthodes RMN de l'$^{27}$Al, fluorescence X et ICP permettent d'accéder aux résultats suivants pour l'IZM-2_B :

- pourcentage pondéral d'atomes d'aluminium hexacoordinés AIVI : 13%,

- rapport du nombre de moles de silicium divisé par le nombre de moles d'aluminium de réseau, en mole/mole, Si/Al : 91,

- rapport du nombre de moles de sodium divisé par le nombre de moles d'aluminium de réseau, en mole/mole, Na/Al : 0,28.

Pour la préparation du catalyseur B on utilise l'échantillon d'alumine imprégnée de platine dont la préparation est décrite dans l'exemple 1. Le catalyseur B est préparé en suivant le même protocole de préparation que pour le catalyseur A de l'exemple 1. La composition du catalyseur B est la suivante :

- 10% poids de la zéolithe IZM-2_B,

- 1,5% poids de platine,

- 88,5% poids d'alumine imprégnée.

Exemple 3 (conforme à l'invention) : préparation du catalyseur d'isomérisation C

Le catalyseur C est un catalyseur comprenant une zéolithe IZM-2, du platine, et une matrice alumine. Cette zéolithe IZM-2 a été synthétisée conformément à l'enseignement du brevet FR 2 918 050 B. Une suspension colloïdale de silice connue sous le terme commercial Ludox HS-40 commercialisée par Aldrich, est incorporée dans une solution composée de soude (Prolabo), de structurant dibromure de 1,6bis(méthylpiperidinium)hexane, d'hydroxyde d'aluminium (Aldrich) et d'eau déionisée. La composition molaire du mélange est la suivante: 1 SiO$_2$; 0,0055 Al$_2$O$_3$ ; 0,1666 Na$_2$O; 0,1666 1,6bis(méthylpiperidinium)hexane; 33,3333 H$_2$O. Le mélange est agité vigoureusement pendant une demi-heure. Le mélange est ensuite transféré, après homogénéisation, dans un autoclave de type PARR. L'autoclave est chauffé pendant 5 jours à 170°C sous agitation en tourne broche (30 tours/min). Le produit obtenu est filtré, lavé à l'eau déionisée pour atteindre un pH neutre puis séché une nuit à 100°C en étuve. Le solide est ensuite introduit dans un four à moufle pour y être calciné afin d'éliminer le structurant. Le cycle de calcination comprend une montée en température jusqu'à 200°C, un palier à cette température de deux heures, une montée en température jusqu'à 550°C suivi d'un palier de huit heures à cette température et enfin un retour à température ambiante. Les montées en température sont effectuées avec une rampe de 2°C/min. Le solide ainsi obtenu est ensuite mis sous reflux durant 2 heures dans une solution aqueuse de nitrate d'ammonium (10 ml de solution par gramme de solide, concentration en nitrate d'ammonium de 3 M) afin d'échanger les cations alcalins sodium par des ions ammonium. Cette étape de mise sous reflux est effectuée quatre fois avec une solution fraiche de nitrate d'ammonium, puis le solide est filtré, lavé à l'eau déionisée et séché en étuve une nuit à 100°C. Enfin, pour obtenir la zéolithe sous sa forme acide (protonée H$^+$) on réalise une étape de calcination à 550°C durant dix heures (rampe de montée en température de 2°C/min) en lit traversé sous air sec (2 normaux litres par heure et par gramme de solide). Le solide ainsi obtenu a été analysé par Diffraction des Rayons X et identifié comme étant constitué par de la zéolithe IZM-2. La zéolithe, nommée IZM-2_C, est utilisée pour la préparation du catalyseur. Des caractérisations au moyen des méthodes RMN de l'$^{27}$Al, fluorescence X et ICP permettent d'accéder aux résultats suivants pour l'IZM-2_C :

- pourcentage pondéral d'atomes d'aluminium hexacoordinés AIVI : 11%,

- rapport du nombre de moles de silicium divisé par le nombre de moles d'aluminium de réseau, en mole/mole, Si/Al : 78,

- rapport du nombre de moles de sodium divisé par le nombre de moles d'aluminium de réseau, en mole/mole, Na/Al : 0,18.

Pour la préparation du catalyseur C on utilise l'échantillon d'alumine imprégnée de platine dont la préparation est décrite dans l'exemple 1. Le catalyseur C est préparé en suivant le même protocole de préparation que pour le catalyseur A de l'exemple 1. La composition du catalyseur C est la suivante :

- 10% poids de la zéolithe IZM-2_C,

- 1,5% poids de platine,

- 88,5% poids d'alumine imprégnée.

Exemple 4 (conforme à l'invention) : préparation du catalyseur d'isomérisation D

Le catalyseur D est un catalyseur comprenant une zéolithe IZM-2, du platine, et une matrice alumine. Cette zéolithe IZM-2 a été synthétisée conformément à l'enseignement du brevet FR 2 918 050 B. Une suspension colloïdale de silice connue sous le terme commercial Ludox HS-40 commercialisée par Aldrich, est incorporée dans une solution composée de soude (Prolabo), de structurant dibromure de 1,6bis(méthylpiperidinium)hexane, d'hydroxyde d'aluminium (Aldrich) et d'eau déionisée. La composition molaire du mélange est la suivante: 1 $SiO_2$; 0,0072 $Al_2O_3$; 0,1666 $Na_2O$; 0,1666 1,6bis(méthylpiperidinium)hexane; 33,3333 $H_2O$. Le mélange est agité vigoureusement pendant une demi-heure. Le mélange est ensuite transféré, après homogénéisation, dans un autoclave de type PARR. L'autoclave est chauffé pendant 5 jours à 170°C sous agitation en tourne broche (30 tours/min). Le produit obtenu est filtré, lavé à l'eau déionisée pour atteindre un pH neutre puis séché une nuit à 100°C en étuve. Le solide est ensuite introduit dans un four à moufle pour y être calciné afin d'éliminer le structurant. Le cycle de calcination comprend une montée en température jusqu'à 200°C, un palier à cette température de deux heures, une montée en température jusqu'à 550°C suivi d'un palier de huit heures à cette température et enfin un retour à température ambiante. Les montées en température sont effectuées avec une rampe de 2°C/min. Le solide ainsi obtenu est ensuite mis sous reflux durant 2 heures dans une solution aqueuse de nitrate d'ammonium (10 ml de solution par gramme de solide, concentration en nitrate d'ammonium de 3 M) afin d'échanger les cations alcalins sodium par des ions ammonium. Cette étape de mise sous reflux est effectuée quatre fois avec une solution fraiche de nitrate d'ammonium, puis le solide est filtré, lavé à l'eau déionisée et séché en étuve une nuit à 100°C. Enfin, pour obtenir la zéolithe sous sa forme acide (protonée $H^+$) on réalise une étape de calcination à 550°C durant dix heures (rampe de montée en température de 2°C/min) en lit traversé sous air sec (2 normaux litres par heure et par gramme de solide). Le solide ainsi obtenu a été analysé par Diffraction des Rayons X et identifié comme étant constitué par de la zéolithe IZM-2. La zéolithe, nommée IZM-2_D, est utilisée pour la préparation du catalyseur. Des caractérisations au moyen des méthodes RMN de l'$^{27}$Al, fluorescence X et ICP permettent d'accéder aux résultats suivants pour l'IZM-2_D :

- pourcentage pondéral d'atomes d'aluminium hexacoordinés AlVI : 12%,

- rapport du nombre de moles de silicium divisé par le nombre de moles d'aluminium de réseau, en mole/mole, Si/Al : 63,

- rapport du nombre de moles de sodium divisé par le nombre de moles d'aluminium de réseau, en mole/mole, Na/Al : 0,19.

Pour la préparation du catalyseur D on utilise l'échantillon d'alumine imprégnée de platine dont la préparation est décrite dans l'exemple 1. Le catalyseur D est préparé en suivant le même protocole de préparation que pour le catalyseur A de l'exemple 1. La composition du catalyseur D est la suivante :

- 10% poids de la zéolithe IZM-2_D,

- 1,5% poids de platine,

- 88,5% poids d'alumine imprégnée.

Exemple 5 (non conforme à l'invention) : préparation du catalyseur d'isomérisation E

Le catalyseur E est un catalyseur comprenant une zéolithe IZM-2, du platine, et une matrice alumine. Cette zéolithe IZM-2 a été synthétisée conformément à l'enseignement du brevet FR 2 918 050 B. Une suspension colloïdale de silice connue sous le terme commercial Ludox HS-40 commercialisée par Aldrich, est incorporée dans une solution composée de soude (Prolabo), de structurant dibromure de 1,6bis(méthylpiperidinium)hexane, d'hydroxyde d'aluminium (Aldrich) et d'eau déionisée. La composition molaire du mélange est la suivante: 1 $SiO_2$; 0,0100 $Al_2O_3$ ; 0,1666 $Na_2O$; 0,1666 1,6bis(méthylpiperidinium)hexane; 33,3333 $H_2O$. Le mélange est agité vigoureusement pendant une demi-heure. Le mélange est ensuite transféré, après homogénéisation, dans un autoclave de type PARR. L'autoclave est chauffé pendant 5 jours à 170°C sous agitation en tourne broche (30 tours/min). Le produit obtenu est filtré, lavé à l'eau déionisée pour atteindre un pH neutre puis séché une nuit à 100°C en étuve. Le solide est ensuite introduit dans un four à moufle pour y être calciné afin d'éliminer le structurant. Le cycle de calcination comprend une montée en température jusqu'à 200°C, un palier à cette température de deux heures, une montée en température jusqu'à 550°C suivi d'un palier de huit heures à cette température et enfin un retour à température ambiante. Les montées en température sont effectuées avec une rampe de 2°C/min. Le solide ainsi obtenu est ensuite mis sous reflux durant 2 heures dans une solution aqueuse de nitrate d'ammonium (10 ml de solution par gramme de solide, concentration en nitrate d'ammonium de 3 M) afin d'échanger les cations alcalins sodium par des ions ammonium. Cette étape de mise sous reflux est effectuée quatre fois avec une solution fraiche de nitrate d'ammonium, puis le solide est filtré, lavé à l'eau déionisée et séché en étuve une nuit à 100°C. Enfin, pour obtenir la zéolithe sous sa forme acide (protonée H⁺) on réalise une étape de calcination à 550°C durant dix heures (rampe de montée en température de 2°C/min) en lit traversé sous air sec (2 normaux litres par heure et par gramme de solide). Le solide ainsi obtenu a été analysé par Diffraction des Rayons X et identifié comme étant constitué par de la zéolithe IZM-2. La zéolithe, nommée IZM-2_E, est utilisée pour la préparation du catalyseur. Des caractérisations par RMN de l'$^{27}$Al, fluorescence X et ICP permettent d'accéder aux résultats suivants pour l'IZM-2_E :

- pourcentage pondéral d'atomes d'aluminium hexacoordinés AlVI : 6%,

- rapport du nombre de moles de silicium divisé par le nombre de moles d'aluminium de réseau, en mole/mole, Si/Al : 46,

- rapport du nombre de moles de sodium divisé par le nombre de moles d'aluminium de réseau, en mole/mole, Na/Al : 0,08.

Pour la préparation du catalyseur E on utilise l'échantillon d'alumine imprégnée de platine dont la préparation est décrite dans l'exemple 1. Le catalyseur E est préparé en suivant le même protocole de préparation que pour le catalyseur A de l'exemple 1. La composition du catalyseur E est la suivante :

- 10% poids de la zéolithe IZM-2_E,

- 1,5% poids de platine,

- 88,5% poids d'alumine imprégnée.

Exemple 6 : évaluation des propriétés catalytiques des catalyseurs A, B, C, D, E, en isomérisation d'une coupe $C_8$ aromatiques

Les catalyseurs ont été testés en isomérisation d'une coupe $C_8$ aromatiques composée par de l'éthylbenzène (19% poids), de l'ortho-xylène (16% poids), du méta-xylène (58% poids) et de l'éthylcyclohexane (7% poids). Les tests ont été effectués dans une micro-unité mettant en oeuvre un réacteur lit fixe et travaillant en courant descendant sans recyclage. L'analyse des effluents hydrocarbonés est effectuée en ligne par chromatographie en phase gazeuse. Avant chargement dans l'unité, le catalyseur est préalablement séché au moins une nuit en étuve à 110°C. Une fois chargé dans l'unité, le catalyseur subit une première étape de séchage sous azote dans les conditions suivantes :

- débit d'azote: 5 normaux litres par heure et par gramme de catalyseur,

- pression totale: 1,3 MPa,

- rampe de montée en température de l'ambiante à 150°C: 10°C/min,

- palier à 150°C de 30 minutes.

Après séchage l'azote est remplacé par l'hydrogène et une étape de réduction sous débit d'hydrogène pur est effectuée ensuite dans les conditions suivantes :

- débit d'hydrogène: 4 normaux litres par heure et par gramme de catalyseur,

- pression totale: 1,3 MPa,

- rampe de montée en température de 150 à 480°C: 5°C/min,

- palier à 480°C de 2 heures.

La température est alors descendue à 425°C puis le catalyseur est stabilisé durant 24 heures sous flux d'hydrogène et d'hydrocarbures (mélange d'éthylbenzène à 20% poids et d'ortho-xylène à 80% poids), dans les conditions opératoires suivantes :

- vitesse spatiale d'alimentation de 5 grammes d'hydrocarbures par heure et par gramme de catalyseur,

- rapport molaire hydrogène sur hydrocarbures de 4,

- pression totale de 1,3 MPa.

Après étape de stabilisation, La température est ensuite descendue à 385°C, et le catalyseur est mis en contact de la coupe $C_8$ aromatiques mentionnée plus haut dans les conditions suivantes :

- vitesse spatiale d'alimentation de 3,5 grammes de la coupe C8 aromatiques par heure et par gramme de catalyseur,

- rapport molaire hydrogène sur hydrocarbures de 4,

- pression totale de 0,86 MPa.

Le catalyseur est maintenu durant 7 heures dans ces conditions opératoires puis les performances catalytiques sont évaluées selon les différentes conditions opératoires qui sont récapitulées dans le Tableau 2 ci-dessous. La variation de la vitesse spatiale d'alimentation permet de faire varier les niveaux de conversion en éthylbenzène et d'isomérisation des xylènes et donc la production de para-xylène. A chaque condition opératoire deux analyses par chromatographie sont effectuées afin de mesurer les performances des catalyseurs.

Tableau 2 : conditions opératoires mises en oeuvre pour l'évaluation catalytique

| conditions | 1 | 2 | 3 | 4 | 5 | 6 |
|---|---|---|---|---|---|---|
| Durée à chaque condition (h) | 2,8 | 2,1 | 2,1 | 2,1 | 2,1 | 2,1 |
| Température (°C) | 385 | 385 | 385 | 385 | 385 | 385 |
| Vitesse spatiale d'alimentation ($h^{-1}$) | 3,5 | 4,5 | 6,0 | 9,0 | 12,0 | 20,0 |
| $H_2$/hydrocarbures (mole/mole) | 4 | 4 | 4 | 4 | 4 | 4 |
| Pression totale (MPa) | 0,86 | 0,86 | 0,86 | 0,86 | 0,86 | 0,86 |

Le rendement en para-xylène (PX) dans l'effluent hydrocarboné obtenu à la vitesse spatiale d'alimentation de 20 $h^{-1}$ permet d'évaluer l'activité des catalyseurs pour la production de para-xylène :

PX = % poids de para-xylène dans l'effluent hydrocarboné,

où PX est le rendement en para-xylène en % poids.

L'évolution du rendement en pertes nettes (PN) en fonction du rendement en para-xylène permet quant à lui d'évaluer la sélectivité du catalyseur. Sont considérées comme pertes nettes toutes les molécules hydrocarbonées autres que les molécules cycliques à huit atomes de carbone :

$$PN = 100 - PX - EB - OX - MX - N8$$

avec :

PN : rendement en pertes nettes dans l'effluent hydrocarboné, en % poids,
PX : % poids de para-xylène dans l'effluent hydrocarboné,
EB : % poids d'éthylbenzène dans l'effluent hydrocarboné,
OX : % poids d'ortho-xylène dans l'effluent hydrocarboné,
MX : % poids de méta-xylène dans l'effluent hydrocarboné,
N8 : % poids des naphtènes à huit atomes de carbone dans l'effluent hydrocarboné.

Le Tableau 3 reporte ainsi le rendement en para-xylène des catalyseurs A, B, C, D et E à une vitesse spatiale de 20 h$^{-1}$ ainsi que les pertes nettes estimées pour un rendement en para-xylène de 18% pour les catalyseurs. Les pertes nettes (PN) à 18% de rendement en para-xylène sont estimées par interpolation ou extrapolation linéaire des données expérimentales de l'évolution du rendement en pertes nettes en fonction du rendement en para-xylène. Les catalyseurs A, B, C, D et E se distinguent uniquement par la nature de la zéolithe IZM-2 entrant dans leur composition.

Le catalyseur E qui met en jeu l'IZM-2_E de rapport Si/Al de 46 est le catalyseur le plus actif de l'ensemble des catalyseurs mais en revanche il est nettement moins sélectif que les autres catalyseurs. Le catalyseur A qui met en jeu l'IZM-2_A de rapport Si/Al de 113 est le solide le moins actif de l'ensemble des catalyseurs alors que sa sélectivité reste comparable à celle des catalyseurs B, C, D. En définitive, l'utilisation des zéolithes IZM-2_B (Si/Al de 91), C (Si/Al de 78) et D (Si/Al de 63) permet d'obtenir une meilleure activité qu'avec la zéolithe IZM-2_A (Si/Al de 113) et une meilleure sélectivité qu'avec la zéolithe IZM-2_E (Si/Al de 46).

Tableau 3 : performances catalytiques des catalyseurs A, B, C, D et E

| Catalyseur | A | B | C | D | E |
|---|---|---|---|---|---|
| Rapport molaire Si/Al (IZM-2) | 113 | 91 | 78 | 63 | 46 |
| Rendement en para-xylène à vitesse spatiale de 20 h$^{-1}$ | 7,6 | 12,6 | 14,7 | 16,6 | 17,7 |
| Rendement en pertes nettes à un rendement en para-xylène de 18% | 2,5 | 2,8 | 2,6 | 2,8 | 3,8 |

## Revendications

1. Procédé d'isomérisation d'une coupe contenant au moins un composé aromatique à huit atomes de carbone par molécule, ledit procédé comprenant la mise en contact de ladite coupe aromatique avec au moins un catalyseur comprenant au moins une zéolithe IZM-2, au moins une matrice et au moins un métal du groupe VIII de la classification périodique des éléments, ledit catalyseur étant **caractérisé en ce que** le rapport entre le nombre de moles de silicium et le nombre de moles d'aluminium de la zéolithe IZM-2 est compris entre 60 et 95
ledit procédé étant mis en oeuvre dans les conditions opératoires suivantes :

   - une température de 300°C à 500°C,
   - une pression partielle d'hydrogène de 0,3 à 1,5 MPa,
   - une pression totale de 0,45 à 1,9 MPa, et
   - une vitesse spatiale d'alimentation, exprimée en kilogramme de charge introduite par kilogramme de catalyseur et par heure, de 0,25 à 30 h$^{-1}$.

2. Procédé selon l'une des revendications précédentes dans lequel la zéolithe IZM-2 du catalyseur mis en œuvre est sous sa forme acide.

3. Procédé selon l'une des revendications précédentes dans lequel ladite matrice du catalyseur mis en œuvre est choisie dans le groupe formé par l'alumine, la silice, la silice-alumine, les argiles, l'oxyde de titane, l'oxyde de bore et la zircone, pris seuls ou en mélange ou bien des aluminates.

**4.** Procédé selon la revendication 3 dans lequel la matrice du catalyseur mis en œuvre est l'alumine.

**5.** Procédé selon l'une des revendications précédentes dans lequel le métal du groupe VIII du catalyseur mis en œuvre est choisi parmi le fer, le cobalt, le nickel, le ruthénium, le rhodium, le palladium, l'osmium, l'iridium et le platine, de préférence parmi les métaux nobles du groupe VIII, de manière très préférée parmi le palladium et le platine, et de manière encore plus préférée le platine.

**6.** Procédé selon l'une des revendications précédentes dans lequel le catalyseur mis en oeuvre comprend au moins un métal additionnel choisi dans le groupe formé par les métaux des groupes IIIA, IVA et VIIB de la classification périodique des éléments et de préférence parmi le gallium, l'indium, l'étain et le rhénium.

**7.** Procédé selon l'une des revendications précédentes dans lequel le catalyseur mis en oeuvre comprend en outre du soufre.

**8.** Procédé selon l'une des revendications précédentes dans lequel le catalyseur mis en oeuvre comprend:

- de 1 à 90% de la zéolithe IZM-2,
- de 0,01 à 4% d'au moins un métal du groupe VIII de la classification périodique des éléments,
- éventuellement de 0,01 à 2% d'au moins un métal additionnel choisi dans le groupe formé par les métaux des groupes IIIA, IVA et VIIB,
- éventuellement une teneur en soufre telle que le rapport du nombre de moles de soufre sur le nombre de moles de(s) métal(ux) du groupe VIII soit compris entre 0,3 et 3,
- au moins une matrice assurant le complément à 100% dans le catalyseur.

**9.** Procédé d'isomérisation selon l'une des revendications précédentes dans lequel ladite coupe aromatique contenant au moins un composé aromatique ayant huit atomes de carbone par molécule comprend comme composé aromatique ayant huit atomes de carbone par molécule soit uniquement un mélange de xylènes, soit uniquement de l'éthylbenzène, soit un mélange de xylène(s) et d'éthylbenzène.

**Patentansprüche**

**1.** Verfahren zur Isomerisierung einer Fraktion, die mindestens eine aromatische Verbindung mit acht Kohlenstoffatomen pro Molekül enthält, wobei im Rahmen des Verfahrens die aromatische Fraktion mit mindestens einem Katalysator in Kontakt gebracht wird, der mindestens einen Zeolith IZM-2, mindestens eine Matrix und mindestens ein Metall der Gruppe VIII des Periodensystems der Elemente umfasst, wobei der Katalysator **dadurch gekennzeichnet ist, dass** das Verhältnis zwischen der Molanzahl an Silicium und der Molanzahl an Aluminium des Zeoliths IZM-2 im Bereich von 60 bis 95 liegt
wobei das Verfahren unter den folgenden Betriebsbedingungen durchgeführt wird:

- einer Temperatur von 300 °C bis 500 °C,
- einem Wasserstoffpartialdruck von 0,3 bis 1,5 MPa,
- einem Gesamtdruck von 0,45 bis 1,9 MPa, und
- einer raumbezogenen Zufuhrgeschwindigkeit, ausgedrückt in Kilogramm an Beschickungsgut, das pro Kilogramm an Katalysator und pro Stunde eingeleitet wird, von 0,25 bis 30 h$^{-1}$.

**2.** Verfahren nach einem der vorhergehenden Ansprüche, wobei der Zeolith IZM-2 des eingesetzten Katalysators in seiner sauren Form vorliegt.

**3.** Verfahren nach einem der vorhergehenden Ansprüche, wobei die Matrix des eingesetzten Katalysators aus der Gruppe ausgewählt ist, die von Aluminiumoxid, Siliciumdioxid, Siliciumdioxid-Aluminiumoxid, Tonsorten, Titanoxid, Boroxid und Zirconiumdioxid, für sich genommen oder in Mischung, oder aber von den Aluminaten gebildet wird.

**4.** Verfahren nach Anspruch 3, wobei es sich bei der Matrix des eingesetzten Katalysators um Aluminiumoxid handelt.

**5.** Verfahren nach einem der vorhergehenden Ansprüche, wobei das Metall der Gruppe VIII des eingesetzten Katalysators aus Eisen, Kobalt, Nickel, Ruthenium, Rhodium, Palladium, Osmium, Iridium und Platin, vorzugsweise aus den Edelmetallen der Gruppe VIII, stark bevorzugt aus Palladium und Platin sowie noch stärker bevorzugt aus Platin ausgewählt ist.

6. Verfahren nach einem der vorhergehenden Ansprüche, wobei der eingesetzte Katalysator mindestens ein zusätzliches Metall umfasst, das aus der Gruppe, welche von den Metallen der Gruppen IIIA, IVA und VIIB des Periodensystems der Elemente gebildet wird, und vorzugsweise aus Gallium, Indium, Zinn und Rhenium ausgewählt ist.

7. Verfahren nach einem der vorhergehenden Ansprüche, wobei der eingesetzte Katalysator darüber hinaus Schwefel umfasst.

8. Verfahren nach einem der vorhergehenden Ansprüche, wobei der eingesetzte Katalysator Folgendes umfasst:

- 1 bis 90 % an Zeolith IZM-2,
- 0,01 bis 4 % mindestens eines Metalls der Gruppe VIII des Periodensystems der Elemente,
- möglicherweise 0,01 bis 2 % mindestens eines zusätzlichen Metalls, das aus der Gruppe ausgewählt ist, welche von den Metallen der Gruppen IIIA, IVA und VIIB gebildet wird,
- möglicherweise einen derartigen Schwefelgehalt, dass das Verhältnis der Molanzahl an Schwefel zur Molanzahl von Metall(en) der Gruppe VIII im Bereich von 0,3 bis 3 liegt,
- mindestens eine Matrix, die im Katalysator den zu 100 % fehlenden Anteil ausmacht.

9. Isomerisierungsverfahren nach einem der vorhergehenden Ansprüche, wobei die aromatische Fraktion, welche mindestens eine aromatische Verbindung mit acht Kohlenstoffatomen pro Molekül enthält, als aromatische Verbindung mit acht Kohlenstoffatomen pro Molekül entweder ausschließlich eine Mischung von Xylolen oder ausschließlich Ethylbenzol oder eine Mischung aus Xylol(en) und Ethylbenzol umfasst.

**Claims**

1. A process for the isomerization of a cut containing at least one aromatic compound containing eight carbon atoms per molecule, said process comprising bring said aromatic cut into contact with at least one catalyst comprising at least one IZM-2 zeolite, at least one matrix and at least one metal from group VIII of the periodic classification of the elements, said catalyst being **characterized in that** the ratio between the number of moles of silicon and the number of moles of aluminium of the IZM-2 zeolite is in the range 60 to 95, said process being carried out under the following operating conditions:

- a temperature of 300°C to 500°C,
- a partial pressure of hydrogen of 0.3 to 1.5 MPa,
- a total pressure of 0.45 to 1.9 MPa, and
- a supply space velocity, expressed in kilograms of feed introduced per kilogram of catalyst and per hour, of 0.25 to 30 $h^{-1}$.

2. The process as claimed in one of the preceding claims, in which the IZM-2 zeolite of the catalyst employed is in its acid form.

3. The process as claimed in one of the preceding claims, in which said catalyst matrix employed is selected from the group formed by alumina, silica, silica-alumina, clays, titanium oxide, boron oxide and zirconia, used alone or as a mixture, or from aluminates.

4. The process as claimed in claim 3, in which the catalyst matrix employed is alumina.

5. The process as claimed in one of the preceding claims, in which the metal from group VIII of the catalyst employed is selected from iron, cobalt, nickel, ruthenium, rhodium, palladium, osmium, iridium and platinum, preferably from noble metals from group VIII, very preferably from palladium and platinum, and more preferably still from palladium.

6. The process as claimed in one of the preceding claims, in which the catalyst employed comprises at least one additional metal selected from the group formed by metals from groups IIIA, IVA and VIIB of the periodic classification of the elements and preferably selected from gallium, indium, tin and rhenium.

7. The process as claimed in one of the preceding claims, in which the catalyst employed further comprises sulfur.

8. The process as claimed in one of the preceding claims, in which the catalyst employed comprises:

   - 1% to 90% of the IZM-2 zeolite,
   - 0.01% to 4% of at least one metal from group VIII of the periodic classification of the elements,
   - optionally 0.01% to 2% of at least one additional metal selected from the group formed by the metals from groups IIIA, IVA and VIIB,
   - optionally a sulfur content such that the ratio of the number of moles of sulfur to the number of moles of metal(s) from group VIII is in the range 0.3 to 3,
   - at least one matrix providing the complement to 100% in the catalyst.

9. The isomerization process as claimed in one of the preceding claims, in which said aromatic cut containing at least one aromatic compound containing eight carbon atoms per molecule comprises, as the aromatic compound containing eight carbon atoms per molecule, either solely a mixture of xylenes, or solely ethylbenzene, or a mixture of xylene(s) and ethylbenzene.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4467129 B
- US 4482773 B
- FR 2477903 B
- EP 923987 A
- WO 2005065380 A
- WO 2010000652 A
- US 20140296601 A

- US 7091190 B
- WO 2010015733 A
- FR 2918050 B
- FR 2934793 B
- EP 0387109 A

**Littérature non-brevet citée dans la description**

- **P. MOREAU.** thèse. l'université de Poitiers, 2005
- **C. MARCILLY.** *catalyse acido-basique,* 2003, vol. 1

- **J. LYNCH.** Analyse physico-chimiques des catalyseurs industriels. 2001, 290, , 291